(19) Europäisches Patentamt
European Patent Office
Office européen des brevets

(11) EP 0 626 364 B1

(12) **FASCICULE DE BREVET EUROPEEN**

(45) Date de publication et mention de la délivrance du brevet:
**08.04.1998 Bulletin 1998/15**

(51) Int. Cl.[6]: **C07C 45/48**, C07C 49/395

(21) Numéro de dépôt: **94401123.8**

(22) Date de dépôt: **20.05.1994**

(54) **Procédé de préparation d'une cétone cyclique**

Verfahren zur Herstellung eines cyclischen Ketons

Process for the preparation of a cyclic ketone

(84) Etats contractants désignés:
**CH DE ES FR GB IT LI**

(30) Priorité: **28.05.1993 FR 9306477**

(43) Date de publication de la demande:
**30.11.1994 Bulletin 1994/48**

(73) Titulaire: **RHONE-POULENC CHIMIE**
**92408 Courbevoie Cédex (FR)**

(72) Inventeurs:
- **Alas, Michel**
  **F-79500 Melle (FR)**
- **Crochemore, Michel**
  **F-69630 Chaponost (FR)**

(74) Mandataire:
**Dutruc-Rosset, Marie-Claude et al**
**RHODIA CHIMIE**
**Direction de la Propriété Industrielle**
**25, quai Paul Doumer**
**92408 Courbevoie Cédex (FR)**

(56) Documents cités:
**EP-A- 0 266 687**
**EP-A- 0 306 873**
**US-A- 3 468 956**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.1/3.4

## Description

La présente invention a pour objet un procédé de préparation d'une cétone cyclique. Plus précisément, l'invention concerne un procédé de préparation d'une cétone cyclique, à partir d'un acide dicarboxylique.

L'invention vise plus particulièrement la préparation de la cyclopentanone à partir de l'acide adipique et de la diméthyl-2,2 cyclopentanone à partir de l'acide diméthyl-2,2 adipique.

Il est connu selon GB-A 615 543 de préparer la cyclopentanone, à partir de l'acide adipique, par chauffage de l'acide adipique, en présence de carbonate ou d'oxyde de manganèse. Le rendement obtenu en cyclopentanone, lorsque la cyclisation est effectuée à 280°C, est excellent, de l'ordre de 90 %.
Toutefois, ce procédé ne donne pas entière satisfaction car il n'est pas possible d'accroître la productivité en cyclopentanone.
En effet, pour maintenir les rendements précités, il est nécessaire de (imiter les débits d'alimentation en acide adipique à environ 0,7 kg/h, pour un kilogramme de catalyseur engagé.
Le débit d'alimentation pourrait être accru en augmentant la température réactionnelle. Bien que la température mentionnée dans la description puisse être choisie dans une gamme de température allant de 280°C à 350°C, il est très difficile d'un point de vue industriel de maintenir une température supérieure à 320°C, sans équipement onéreux ni fluides calorifiques spéciaux et chers.
De plus, il est très difficile, sinon impossible, de maintenir une telle température de manière homogène, dans un tel milieu visqueux constitué de l'acide adipique liquide et du catalyseur à base de manganèse. En outre, il peut exister en plus des problèmes évoqués précédemment, des risques de dépôt de substances polymériques sur le réacteur (phénomène de blindage) qui sont difficiles à éliminer.

Il est également décrit dans US-A 2 612 524, un procédé de préparation de cyclopentanone par cyclisation de l'acide adipique, en présence d'une catalyseur qui est un oxyde de magnésium. Ce procédé requiert aussi une température élevée puique celle-ci se situe entre 300°C et 350°C.

Par ailleurs, il a été décrit dans EP-A-0 266 687 un procédé de préparation de la cyclopentanone à partir d'ester adipique, en présence d'un catalyseur zéolithique et/ou phosphate, en phase gazeuse ou liquide, à une température comprise entre 150°C et 450°C.

Par rapport aux procédés décrits dans l'état de la technique, l'objectif de la présente invention est de disposer d'un procédé permettant d'accroître la productivité en cyclopentanone tout en étant aisément mis en oeuvre à l'échelle industrielle, sans faire appel à un appareillage sophistiqué.

Il a maintenant été trouvé et c'est ce qui consitue l'objet de la présente invention, un procédé de préparation d'une cétone cyclique à partir d'un acide dicarboxylique correspondant ayant une condensation en atomes suffisante pour former le cycle caractérisé par le fait que l'on conduit la réaction, en phase liquide, en présence d'une quantité efficace d'un phosphate neutre condensé ou non.

Par "phosphate neutre", on entend un composé du phosphore qui est dérivé de l'acide orthophosphorique et dont les protons sont subtitués par un cation métallique, ammonium ou autre.

Ainsi, les catalyseurs de l'invention, à savoir les phosphates neutres, et de préférence, le phosphate de sodium étant plus actifs que les catalyseurs à base de manganèse, il est possible selon l'invention d'augmenter le débit de l'acide dicarboxylique et plus particulièrement de l'acide adipique, dans un rapport de 1 à 4.

Conformément au procédé de l'invention, on met en oeuvre un acide dicarboxylique qui répond plus particulièrement à la formule (I) suivante :

$$HOOC - R - COOH \qquad (I)$$

dans ladite formule (I), R représente un radical divalent, substitué ou non, comprenant un enchaînement linéaire d'atomes en un nombre suffisant pour former le cycle cétonique désiré.

Généralement, le radical R comprend un enchaînement linéaire de 2 à 10 atomes et, de préférence, de 2 à 7 atomes et encore plus préférentiellement de 4 ou 5. Il s'agit le plus souvent d'un enchaînement d'atomes de carbone mais l'invention n'exclut pas que la chaîne hydrocarbonée soit interrompue par un hétéroatome, notamment azote, oxygène ou soufre.

Comme mentionné précédemment, le radical R divalent peut être substitué c'est-à-dire que les atomes d'hydrogène de la chaîne hydrocarbonée peuvent être remplacés par un groupe ou une fonction organique. N'importe quels substituants peuvent être présents dans la mesure où ceux-ci n'interfèrent pas au niveau de la réaction de cyclisation. En particulier, la chaîne hydrocarbonée peut porter des chaînes latérales ou ramifications qui peuvent être constituées de préférence par des radicaux alkyle qui, généralement, ont de 1 à 4 atomes de carbone. Les ramifications se situent le plus souvent sur un ou les deux atomes de carbone en position $\alpha$ ou $\beta$ des groupes carboxyliques.

D'une manière gobale, le radical R présente une condensation totale en carbone pouvant varier largement de 2 atomes de carbone jusqu'à un nombre pouvant s'élever à 40 atomes de carbone lorsqu'il y a présence de substituants

et ledit radical comprend un enchaînement linéaire de 2 à 10 atomes qui intervient ensuite dans le cycle obtenu.

Dans la formule (I), R représente, de préférence, un radical divalent aliphatique saturé ou insaturé, linéaire ou ramifié.

Plus précisément, R représente un radical aliphatique saturé linéaire ou ramifié ayant de préférence de 2 à 20 atomes de carbone ou un radical insaturé, linéaire ou ramifié, comprenant une à plusieurs insaturations sur la chaîne, généralement, 1 à 3 insaturations qui peuvent être de préférence, des doubles liaisons simples ou conjuguées.

Conviennent tout particulièrement bien à la mise en oeuvre du procédé de l'invention, les acides dicarboxyliques de formule générale (I) dans laquelle le radical aliphatique R est un radical alkylène linéaire ou ramifié ayant de 2 à 12 atomes de carbone qui comprend un enchaînement linéaire de 2 à 8 atomes de carbone entre les deux groupes COOH.

Le radical R préféré comprend un enchaînement linéaire de 4 ou 5 atomes de carbone entre les deux groupes COOH.

Il est également possible de faire appel dans le procédé de l'invention, à un acide dicarboxylique de formule (I) dans laquelle deux atomes de carbone vicinaux de l'enchaînement linéaire du radical R font partie d'un cycle.

Par cycle, on entend un cycle carbocyclique ou hétérocyclique, saturé, insaturé ou aromatique.

Comme exemples de cycles, on peut envisager des cycles cycloaliphatiques, aromatiques ou hétérocycliques, notamment cycloalkyles comprenant 6 atomes de carbone dans le cycle ou benzéniques, ces cycles pouvant être eux-mêmes éventuellement porteurs de un ou plusieurs substituants dans la mesure où ils n'interfèrent pas avec la réaction de cyclisation.

Comme exemples de tels radicaux R, on peut mentionner, entre autres, les radicaux suivants :

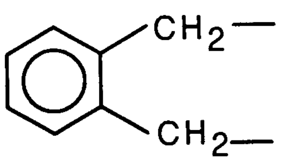 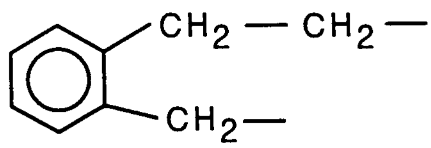

A titre d'acides carboxyliques de formule (I) convenant à la présente invention, on fait appel plus particulièrement aux acides dicarboxyliques suivants :

- l'acide adipique,
- l'acide méthyl-2 adipique
- l'acide méthyl-3 adipique
- l'acide méthyl-4 adipique
- l'acide méthyl-5 adipique
- l'acide diméthyl-2,2 adipique
- l'acide diméthyl-3,3 adipique
- l'acide triméthyl-2,2,5 adipique
- l'acide diméthyl-2,5 adipique
- l'acide pimélique (heptanedioïque)
- l'acide méthyl-2 pimélique
- l'acide diméthyl-2,2 pimélique
- l'acide diméthyl-3,3 pimélique
- l'acide diméthyl-2,5 pimélique
- l'acide triméthyl-2,2,5 pimélique
- l'acide azélaïque
- l'acide sébacique
- l'acide phénylène-1,2 diacétique

Conformément au procédé de l'invention, on effectue la réaction de cyclisation de l'acide dicarboxylique, en présence d'un catalyseur.

Selon le procédé de l'invention, on peut engager le phosphate, sous n'importe quelle forme. Toutefois, pour des raisons économiques, il est préférable d'avoir recours aux formes commerciales facilement accessibles.

On fait appel de préférence aux orthophosphates mais il est également possible de mettre en oeuvre des phosphates condensés c'est-à-dire des phosphates contenant plus d'un atome de phosphore. Ils sont formés d'un enchaînement de motifs tétraédriques $PO_4$ unis entre eux par des atomes d'oxygène. Ces motifs peuvent former par exemple

des chaînes de polyphosphates linéaires contenant de 2 à 10 atomes de phosphore. Comme exemples spécifiques, on peut citer les anions respectivement avec 2 ou 3 atomes de phosphore tels que pyrophosphate $P_4O_7^{2-}$ ou tripolyphosphate $P_3O_{10}^{5-}$.

En ce qui concerne le contre-anion, il peut être de nature quelconque. Il peut s'agir d'un élément métallique et plus particulièrement un élément du groupe la, 2a ou 3b de la Classification périodique des éléments ou bien un cation ammonium.

Pour la définition des éléments, on se réfère ci-après à la Classification périodique des éléments publiée dans le Bulletin de la Société Chimique de France, n°1 (1966).

Le catalyseur mis en oeuvre est un phosphate métallique ou d'ammonium. Il convient également de faire appel à des mélanges de phosphates ou à leurs sels mixtes.

Comme exemples spécifiques de phosphates convenant tout à fait bien à l'invention, on peut mentionner :

- le phosphate de sodium $Na_3PO_4$,
- le phosphate de potassium,
- le pyrophosphate de sodium $Na_2P_4O_7$,
- le pyrophosphate de potassium,
- le phosphate d'aluminium,
- le phosphate d'ammonium,
- le phosphate d'argent,
- le phosphate de baryum,
- le phosphate de calcium,
- le phosphate de chrome,
- le phosphate de cobalt,
- le phosphate de cuivre,
- le phosphate double de magnésium et d'ammonium,
- le phosphate de fer,
- le phosphate ferreux,
- le phosphate de lithium,
- le phosphate de magnésium,
- le phosphate de manganèse,
- le phosphate de potassium,
- le phosphate de zinc,
- le pyrophosphate de calcium,
- le pyrophosphate de cuivre
- le pyrophosphate de zinc,
- le pentapolyphosphate de sodium $Na_7P_5O_{16}$,
- le tripolyphosphate de sodium $Na_5P_3O_{10}$,
- le tripolyphosphate de potassium $K_5P_3O_{10}$.

Le phosphate intervenant dans le procédé de l'invention peut être mis en oeuvre sous forme anhydre ou hydratée.

Parmi tous les catalyseurs cités, les catalyseurs choisis préférentiellement sont les phosphates, les pyrophosphates, les tripolyphosphates ou les pentapolyphosphate de sodium ou de potassium.

Les catalyseurs de l'invention étant actifs à basse température, le procédé de l'invention est mis en oeuvre en phase liquide, de préférence, en présence d'un solvant réactionnel.

L'acide dicarboxylique peut être utilisé en tant que solvant réactionnel mais d'une manière préférentielle, on fait appel à un solvant organique en tant que flux de transfert thermique.

Plusieurs impératifs président au choix du solvant organique.

Il doit être stable dans les conditions réactionnelles et inerte vis-à-vis de l'acide dicarboxylique de départ et de la cétone cyclique obtenue.

Il doit présenter une température d'ébullition élevée, de préférence comprise entre 200°C et 500°C.

Comme exemples de solvants convenant particulièrement à l'invention, on peut mentionner entre autres :

- les hydrocarbures aliphatiques et/ou aromatiques et plus particulièrement les paraffines tels que notamment, le décane, le undécane, le dodécane ou le tétradécane; les hydrocarbures aromatiques comme notamment les xylènes, le cumène, les coupes pétrolières constituées de mélange d'alkylbenzènes notamment les coupes de type Solvesso®,
- les esters lourds d'acides minéraux (par exemple, le phosphate de tricrésyle) ou d'acides carboxyliques (par exemple, le phtalate d'octyle),

- les éthers et plus particulièrement les éthers aromatiques comme l'oxyde de biphényle et/ou l'oxyde de benzyle,
- les huiles paraffiniques et/ou naphténiques, résidus de distillation du pétrole.

On peut également mettre en oeuvre un mélange de solvants organiques.

Interviennent donc dans le procédé de l'invention, l'acide dicarboxylique de départ, le catalyseur de la réaction et le solvant organique.

La concentration de l'acide dicarboxylique dans la masse réactionnelle peut varier largement. Généralement, l'acide dicarboxylique représente de 20 à 50 % du poids de la masse réactionnelle.

La quantité de catalyseur mise en oeuvre, exprimée en nombre d'atomes de cation métallique pour 100 moles d'acide dicarboxylique peut être avantageusement comprise entre 0,1 et 20 %, de préférence entre 1 et 10 %.

D'un point de vue pratique, lorsque le procédé de l'invention est mis en oeuvre en discontinu, généralement, on charge d'abord le solvant réactionnel et le catalyseur puis l'on ajoute l'acide dicarboxylique, de préférence fondu préalablement.

Le procédé de l'invention peut être mis en oeuvre aussi bien en discontinu qu'en continu. Dans ce cas-là, seul est alimenté l'acide dicarboxylique.

Le débit d'alimentation de l'acide dicarboxylique peut varier largement, entre 0,1 et 4,0 kg/heure pour un kilogramme de catalyseur introduit. Il est choisi préférentiellement entre 0,5 et 1,0 kg/heure et par kilogramme de catalyseur.

Le procédé de l'invention est avantageusement conduit à une température inférieure à 300°C, de préférence comprise entre 200°C et 300°C et encore plus préférentiellement entre 250°C et 290°C.

Il est généralement mis en oeuvre sous pression atmosphérique mais également sous pression réduite comprise par exemple entre 50 et 760 mm de mercure.

Une variante préférée du procédé de l'invention consiste à éliminer par distillation, au fur et à mesure de leur formation, la cétone cyclique, le gaz carbonique et l'eau formés.

En fin de réaction, on récupère la cétone cyclique à partir du distillat, selon les techniques classiques utilisées dans ce domaine technique, notamment par décantation ou par cristallisation.

Le procédé de l'invention est tout à fait bien adapté à la préparation de la cyclopentanone, de la diméthyl-2,2 cyclopentanone et de la cyclohexanone.

Les exemples qui suivent, illustrent l'invention sans toutefois la limiter.

Dans les exemples, les abréviations suivantes signifient :

RAH = rapport d'alimentation horaire en acide adipique par rapport au catalyseur.

$$RAH = \frac{\text{charge horaire en acide adipique (en poids)}}{\text{charge en catalyseur (en poids)}}$$

$$TT = \frac{\text{nombre de moles d'acide adipique transformées}}{\text{nombre de moles d'acide adipique introduites}}\ \%$$

$$RT = \frac{\text{nombre de moles de cyclopentanone formées}}{\text{nombre de moles d'acide adipique transformées}}\ \%$$

## EXEMPLES

### Exemples 1 à 4 :

Les exemples suivants sont conduits selon un mode de réalisation en continu.

On donne, ci-après, le protocole opératoire qui va être suivi dans tous les exemples.

L'appareillage utilisé est toujours le même. Il s'agit d'un ballon en verre de 1000 ml, muni d'une agitation magnétique, surmonté d'une colonne Raschig de 20 mm de diamètre et de 100 mm de hauteur. La tête de colonne comprend une ampoule de coulée chauffée avec un pistolet à air chaud pour alimenter l'acide adipique qui est préalablement fondu.

On charge le solvant réactionnel et le catalyseur puis l'on ajoute l'acide adipique fondu.

On effectue une distillation en continu de la cyclopentanone à 130°C en tête, en alimentant l'acide adipique sur le mélange solvant/catalyseur maintenu à une température de 250°C.

Les différentes quantités d'acide adipique et de catalyseur chargées sont indiquées dans le tableau récapitulatif qui suit.

La nature du solvant réactionnel est précisée dans ledit tableau. Le volume de solvant engagé est de 500ml.

En fin de réaction, on récupère un distillat comprenant de l'eau et la cyclopentanone. On sépare l'eau de la cyclopentanone en saturant le distillat par du chlorure de sodium. La cyclopentanone est dosée par chromatographie en phase gazeuse.

Quant au culot de distillation, il est extrait par 3 x 600 ml d'un mélange d'eau et d'hydroxyde de sodium (60/40 en volume). Le volume total est ajusté à 2000 ml. On prélève 10 ml ajusté à 100 ml avec un mélange eau/hydroxyde de sodium (15/85 en volume) à 0,035 % d'acide phosphorique. On dose l'acide adipique non transformé, par chromatographie liquide, haute performance.

Les essais sont conduits selon le protocole opératoire précédemment défini.

Toutes les conditions opératoires et résultats obtenus sont consignés dans le tableau (I) suivant :

Tableau (I)

| Ref. | Solvant | | Catalyseur | | Acide adipique chargé | | RAH | Temps (h) | Acide restant (g) | Cyclo-pentanone | RT (%) |
|---|---|---|---|---|---|---|---|---|---|---|---|
| | Nature | Volume | Nature | Poids | Total (g) | /h | | | | Poids (g) | |
| 1 | oxyde de biphényle | 500 | $Na_3PO_4{\cdot}H_2O$ | 8,6 | 475 | 19,8 | 2,3 | 24 | 90 | 204 | 92 |
| 2 | oxyde de benzyle | 500 | $FePO_4,2H_2O$ | 9,5 | 300 | 18,75 | 2,0 | 16 | 80 | 83 | 66 |
| 3 | oxyde de biphényle<br>oxyde de benzyle | 450<br>50 | $BiPO_4$ | 8 | 217,5 | 27,2 | 3,4 | 8 | 52 | 56,6 | 59,5 |
| 4 | oxyde de biphényle | 500 | $LiPO_4$ | 9 | 500 | 20,8 | 2,3 | 24 | 45 | 246 | 94 |

Exemples 5 à 9 :

On utilise le protocole opératoire défini ci-après.

On réalise une série d'essais en discontinu.

Dans un réacteur de 250 ml muni d'une agitation magnétique et surmonté d'une colonne garnie Multiknit de 20 mm de diamètre et 100 mm de hauteur, calorifugée et équipée d'une tête de colonne, on charge :

- le solvant réactionnel qui est l'oxyde de biphényle à raison de 140 ml,
- le catalyseur dont la nature est précisée dans le tableau (II) à raison de 0,01 mol,
- l'acide adipique fondu, soit 0,2 mol (29,2 g).

On porte le mélange réactionnel, à léger reflux de façon que les vapeurs ne dépassent pas le bas de la colonne.

La cyclopentanone formée est distillée en tête de colonne à une température égale à 130°C jusqu'à épuisement.

La durée de l'opération est de 8 heures.

Les résultats obtenus sont rassemblés dans le tableau (II).

Tableau (II)

| Ref. | Catalyseur | | TT (%) | RT (%) |
|---|---|---|---|---|
| | Nature | Poids | | |
| 5 | $Na_3PO_4$, $H_2O$ | 1,8 | 71 | 90 |
| 6 | $Fe_3(PO_4)_2,8H_2O$ | 5,0 | 82 | 93 |
| 7 | $Mn_3(PO_4)_2,3H_2O$ | 4,1 | 59 | 67 |
| 8 | $Pb_3(PO_4)_2$ | 8,1 | 64 | 72 |
| 9 | $K_3PO_4,2H_2O$ | 2,5 | 70 | 85 |

Exemple 10 :

On chauffe 5 g d'acide diméthyl-2,2 adipique, en présence de 1,1 g de phosphate de sodium $Na_3PO_4$, $H_2O$ et on ajoute 17 ml d'oxyde de biphényle à 225°C en 1 heure 35.

Le distillat refroidi vers 0°C, est biphasique. Après élimination de l'eau par absorption sur sulfate de sodium, la diméthyl-2,2 cyclopentanone obtenue est dosée par chromatographie en phase gazeuse.

Le résidu de chaudière est traité par des lavages aqueux basiques (3 x 100 ml d'une solution aqueuse de soude 1 N) pour extraire l'acide non transformé.

Le dosage, par chromatographie liquide haute performance, conduit aux résultats suivants :

- $TT_{\text{acide adipique}}$ = 85 %,
- $RT_{\text{diméthyl-2,2 cyclopentanone}}$ = 90 %.

**Revendications**

1. Procédé de préparation d'une cétone cyclique à partir d'un acide dicarboxylique correspondant ayant une condensation en atomes suffisante pour former le cycle caractérisé par le fait que l'on conduit la réaction, en phase liquide, en présence d'une quantité efficace d'un phosphate neutre condensé ou non.

2. Procédé selon la revendication 1 caractérisé par le fait que l'acide dicarboxylique mis en oeuvre répond à la formule (I) suivante :

$$HOOC\text{-}R\text{-}COOH \qquad (I)$$

dans ladite formule (I), R représente un radical divalent, substitué ou non, comprenant un enchaînement linéaire d'atomes en un nombre suffisant pour former le cycle cétonique désiré.

3. Procédé selon l'une des revendications 1 et 2 caractérisé par le fait que l'acide dicarboxylique mis en oeuvre

répond à la formule (I) dans laquelle le radical R comprend un enchaînement linéaire de 2 à 10 atomes et, de préférence, de 2 à 7 atomes et encore plus préférentiellement de 4 ou 5.

4. Procédé selon l'une des revendications 1 à 3 caractérisé par le fait que l'acide dicarboxylique mis en oeuvre répond à la formule (I) dans laquelle le radical R divalent est substitué de telle sorte que la chaîne hydrocarbonée porte des chaînes latérales ou ramifications qui sont constituées par des radicaux alkyle ayant de 1 à 4 atomes de carbone ; lesdites ramifications se situant sur un ou les deux atomes de carbone en position $\alpha$ ou $\beta$ des groupes carboxyliques.

5. Procédé selon l'une des revendications 1 à 4 caractérisé par le fait que l'acide dicarboxylique mis en oeuvre répond à la formule (I) dans laquelle le radical R présente une condensation totale en carbone variant de 2 à 40 atomes de carbone et comprend un enchaînement linéaire de 2 à 10 atomes qui intervient ensuite dans le cycle obtenu.

6. Procédé selon l'une des revendications 1 à 5 caractérisé par le fait que l'acide dicarboxylique mis en oeuvre répond à la formule (I) dans laquelle le radical R représente un radical divalent aliphatique saturé ou insaturé, linéaire ou ramifié.

7. Procédé selon l'une des revendications 1 à 6 caractérisé par le fait que l'acide dicarboxylique mis en oeuvre répond à la formule (I) dans laquelle le radical aliphatique R est un radical alkylène linéaire ou ramifié ayant de 2 à 12 atomes de carbone qui comprend un enchaînement linéaire de 2 à 8 atomes de carbone entre les deux groupes COOH et, de préférence, de 4 à 5 atomes de carbone entre les deux groupes COOH.

8. Procédé selon l'une des revendications 2 à 5 caractérisé par le fait que l'acide dicarboxylique mis en oeuvre répond à la formule (I) dans laquelle deux atomes de carbone vicinaux de l'enchaînement linéaire du radical R font partie d'un cycle carbocyclique ou hététocyclique, saturé, insaturé ou aromatique.

9. Procédé selon l'une des revendications 1 à 8 caractérisé par le fait que l'acide dicarboxylique de formule (I) mis en oeuvre est choisi parmi :

- l'acide adipique,
- l'acide méthyl-2 adipique
- l'acide méthyl-3 adipique
- l'acide méthyl-4 adipique
- l'acide méthyl-5 adipique
- l'acide diméthyl-2,2 adipique
- l'acide diméthyl-3,3 adipique
- l'acide triméthyl-2,2,5 adipique
- l'acide diméthyl-2,5 adipique
- l'acide pimélique (heptanedioïque)
- l'acide méthyl-2 pimélique
- l'acide diméthyl-2,2 pimélique
- l'acide diméthyl-3,3 pimélique
- l'acide diméthyl-2,5 pimélique
- l'acide triméthyl-2,2,5 pimélique
- l'acide azélaïque
- l'acide sébacique
- l'acide phénylène-1,2 diacétique

10. Procédé selon l'une des revendications 1 à 9 caractérisé par le fait que l'acide dicarboxylique de formule (I) mis en oeuvre est l'acide adipique ou l'acide diméthyl-2,2 adipique

11. Procédé selon l'une des revendications 1 à 10 caractérisé par le fait que le catalyseur mis en oeuvre est un phosphate, un pyrophosphate ou un polyphosphate.

12. Procédé selon la revendication 11 caractérisé par le fait que le contre-anion est un cation métallique et de préférence, un cation d'un élément du groupe la, 2a ou 3b de la Classification périodique ou bien un cation ammonium.

13. Procédé selon l'une des revendications 1 à 12 caractérisé par le fait que le catalyseur mis en oeuvre est choisi

parmi les phosphates, les pyrophosphates, les tripolyphosphates ou les pentapolyphosphate de sodium ou de potassium.

**14.** Procédé selon l'une des revendications 1 à 13 caractérisé par le fait que la réaction est conduite en phase liquide, en présence d'un solvant réactionnel.

**15.** Procédé selon la revendication 14 caractérisé par le fait que le solvant réactionnel est choisi parmi :

- les hydrocarbures aliphatiques et/ou aromatiques et plus particulièrement les paraffines tels que notamment, le décane, le undécane, le dodécane ou le tétradécane; les hydrocarbures aromatiques comme notamment les xylènes, le cumène, les coupes pétrolières constituées de mélange d'alkylbenzènes notamment les coupes de type Solvesso®,
- les esters lourds d'acides minéraux (par exemple, le phosphate de tricrésyle) ou d'acides carboxyliques (par exemple, le phtalate d'octyle),
- les éthers et plus particulièrement les éthers aromatiques comme l'oxyde de biphényle et/ou l'oxyde de benzyle,
- les huiles paraffiniques et/ou naphténiques, résidus de distillation du pétrole.

**16.** Procédé selon l'une des revendications 1 à 15 caractérisé par le fait que la concentration de l'acide dicarboxylique dans la masse réactionnelle représente de 20 à 50 % du poids de la masse réactionnelle.

**17.** Procédé selon l'une des revendications 1 à 16 caractérisé par le fait que la quantité de catalyseur mise en oeuvre, exprimée en nombre d'atomes de cation métallique pour 100 moles d'acide dicarboxylique est comprise entre 0,1 et 20 %, de préférence entre 1 et 10%.

**18.** Procédé selon l'une des revendications 1 à 17 caractérisé par le fait que l'on charge d'abord le solvant réactionnel et le catalyseur puis l'on ajoute l'acide dicarboxylique, de préférence fondu préalablement.

**19.** Procédé selon l'une des revendications 1 à 18 caractérisé par le fait que le débit d'alimentation de l'acide dicarboxylique varie entre 0,1 et 4,0 kg/heure pour un kilogramme de catalyseur introduit; de préférence, entre 0,5 et 1,0 kg/heure et par kilogramme de catalyseur.

**20.** Procédé selon l'une des revendications 1 à 19 caractérisé par le fait que la température réactionnelle est comprise entre 200 et 300°C, de préférence entre 250°C et 290°C.

## Claims

**1.** A process for the preparation of a cyclic ketone from a corresponding dicarboxylic acid having a condensation of atoms which is sufficient to form the ring characterised in that the reaction is carried out in the liquid phase in the presence of an effective quantity of a condensed or uncondensed neutral phosphate.

**2.** A process according to claim 1 characterised in that the dicarboxylic acid used corresponds to following formula (I):

$$HOOC - R - COOH \qquad (I)$$

in which formula (I) R represents a substituted or unsubstituted divalent radical comprising a straight chain of atoms in a sufficient number to form the desired ketonic ring.

**3.** A process according to one of claims 1 and 2 characterised in that the dicarboxylic acid used corresponds to the formula (I) in which the radical R comprises a straight chain of from 2 to 10 atoms and preferably from 2 to 7 atoms and still are preferably 4 or 5 atoms.

**4.** A process according to one of claims 1 to 3 characterised in that the dicarboxylic acid used corresponds to the formula (I) in which the divalent radical R is substituted in such a way that the hydrocarbon chain bears lateral or branch chains which are formed by alkyl radicals having from 1 to 4 carbon atoms; the branch chains beingpreferably disposed on one or the two carbon atoms in the position $\alpha$ or $\beta$ of the carboxylic groups.

**5.** A process according to one of clams 1 to 4 characterised in that the dicarboxylic acid used corresponds to the formula (I) in which the radical R has a total condensation of carbon varying from 2 to 40 carbon atoms and comprises a straight chain of from 2 to 10 atoms which are then involved in the ring produced.

**6.** A process according to one of claims 1 to 5 characterised in that the dicarboxylic acid used corresponds to the formula (I) in which the radical R represents a saturated or unsaturated, branched or straight chain aliphatic divalent radical.

**7.** A process according to one of claims 1 to 6 characterised in that the dicarboxylic acid used corresponds to the formula (I) in which the aliphatic radical R is a branched or straight chain alkylene radical having from 2 to 12 carbon atoms which comprises a straight chain of from 2 to 8 carbon atoms between the two groups COOH and preferably from 4 to 5 carbon atoms between the two groups COOH.

**8.** A process according to one of claims 2 to 5 characterised in that the dicarboxylic acid used corresponds to the formula (I) in which two adjacent carbon atoms of the straight chain of the radical R form part of a saturated, unsaturated or aromatic, carboxylic or heterocyclic ring.

**9.** A process according to one of claims 1 to 8 characterised in that the dicarboxylic acid of formula (I) used is selected from:

- adipic acid,
- 2-methyl adipic acid,
- 3-methyl adipic acid,
- 4-methyl adipic acid,
- 5-methyl adipic acid,
- 2,2-dimethyl adipic acid,
- 3,3-dimethyl adipic acid,
- 2,2,5-trimethyl adipic acid,
- 2,5-dimethyl adipic acid,
- pimelic (heptanedioic) acid,
- 2-methyl pimelic acid,
- 2,2-dimethyl pimelic acid,
- 3,3-dimethyl pimelic acid,
- 2,5-dimethyl pimelic acid,
- 2,2,5-trimethyl pimelic acid,
- azelaic acid,
- sebacic aacid, and
- 1,2-phenylene diacetic acid.

**10.** A process according to one of claims 1 to 9 characterised in that the dicarboxylic acid of formula (I) used is adipic acid or 2,2-dimethyl adipic acid.

**11.** A process according to one of claims 1 to 10 characterised in that the catalyst used is a phosphate, a pyrophosphate or a polyphosphate.

**12.** A process according to claim 11 characterised in that the counter-anion is a metallic cation and preferably a cation of an element of group 1a, 2a or 3b of the period table or an ammonium cation.

**13.** A process according to one of claims 1 to 12 characterised in that the catalyst used is selected from sodium or potassium phosphates, pyrophosphates, tripolyphosphates or pentapolyphosphates.

**14.** A process according to one of claims 1 to 13 characterised in that the reaction is carried out in the liquid phase, in the presence of a reaction solvent.

**15.** A process according to claim 14 characterised in that the reaction solvent is selected from:

- aliphatic and/or aromatic hydrocarbons and are particularly paraffins such as in particular decane, undecane, dodecane or tetradecane; aromatic hydrocarbons such as in particular xylenes, cumene, and petroleum cuts

formed by a mixture of alkyl benzenes, in particular cuts of Solvesso® type,
- heavy esters of mineral acids (for example tricresyl phosphate) or carboxylic acids (for example octylphthalate),
- ethers and more particularly aromatic ethers such as biphenyl oxide and/or benzyl oxide, and
- paraffinic and/or naphthenic oils, petroleum distillation residues.

**16.** A process according to one of claims 1 to 15 characterised in that the concentration of dicarboxylic acid in the reaction mass represents from 20 to 50% of the weight of the reaction mass.

**17.** A process according to one of claims 1 to 16 characterised in that the amount of catalyst used, expressed in terms of the number of atoms of metallic cation for 100 moles of dicarboxylic acid, is between 0.1 and 20%, preferably between 1 and 10%.

**18.** A process according to one of claims 1 to 17 characterised in that first the reaction solvent and the catalyst are introduced and then the dicarboxylic acid which has preferably been melted beforehand is added.

**11.** A process according to one of claims 1 to 18 characterised in that the dicarboxylic acid feed flow rate varies between 0.1 and 4.0 kg/hour for a kilogram of catalyst introduced and preferably between 0.5 and 1.0 kg/hour and per kilogram of catalyst.

**20.** A process according to one of claims 1 to 19 characterised in that the reaction temperature is between 200 and 300° C, preferably between 250°C and 290°C.

**Patentansprüche**

1. Verfahren zur Herstellung eines ringförmigen (cyclischen) Ketons ausgehend von einer entsprechenden Dicarbonsäure mit einer ausreichenden Kondensation an Atomen zur Ausbildung eines Ringes, dadurch gekennzeichnet, daß man die Reaktion in flüssiger Phase in Gegenwart einer wirksamen Menge eines gegebenenfalls kondensierten, neutralen Phosphats durchführt.

2. Verfahren nach Anspruch 1, dadurch gekennzeichnet, daß die eingesetzte Dicarbonsäure der folgenden Formel (I) entspricht HOOC-R-COOH (I) in der R einen zweiwertigen (divalenten), gegebenenfalls substituierten Rest darstellt, der eine lineare Kette von Atomen in einer ausreichenden Anzahl für die Bildung des gewünschten Ketonringes umfaßt.

3. Verfahren nach Anspruch 1 oder 2, dadurch gekennzeichnet, daß die eingesetzte Dicarbonsäure der Formel (I) entspricht, in der der Rest R eine lineare Kette mit 2 bis 10 Atomen, vorzugsweise mit 2 bis 7 Atomen, insbesondere mit 4 oder 5 Atomen, umfaßt.

4. Verfahren nach einem der Ansprüche 1 bis 3, dadurch gekennzeichnet, daß die eingesetzte Dicarbonsäure der Formel (I) entspricht, in der der zweiwertige Rest R derart substituiert ist daß die Kohlenwasserstoffkette Seitenketten oder Verzweigungen enthält, die aus Alkylresten mit 1 bis 4 Kohlenstoffen bestehen, wobei sich diese Verzweigungen an einen, oder zwei Atomen in $\alpha$-Position oder $\beta$-Position zu den Carbonylgruppen befinden.

5. Verfahren nach einen, der Ansprüche 1 bis 4, dadurch gekennzeichnet, daß die eingesetzte Dicarbonsäure der Formel (I) entspricht, in der der Rest R einen Gesamtkondensationsgrad in bezug auf Kohlenstoff zwischen 2 bis 40 Kohlenstoffatomen aufweist und eine lineare Kette mit 2 bis 10 Atomen umfaßt, die anschließend in dem erhaltenen Ring enthalten ist.

6. Verfahren nach einem der Ansprüche 1 bis 5, dadurch gekennzeichnet, daß die eingesetzte Dicarbonsäure der Formel (I) entspricht, in der der Rest R einen linearen oder verzweigten, gesättigten oder ungesättigten, zweiwertigen aliphatischen Rest darstellt.

7. Verfahren nach einem der Ansprüche 1 bis 6, dadurch gekennzeichnet, daß die eingesetzte Dicarbonsäure der Formel (I) entspricht, in der der aliphatische Rest R ein linearer oder verzweigter Alkylenrest mit 2 bis 12 Kohlenstoffatomen ist, der eine lineare Kette mit 2 bis 8 Kohlenstoffatomen zwischen den zwei COOH-Gruppen, vorzugsweise mit 4 bis 5 Kohlenstoffatomen zwischen den zwei COOH-Gruppen, enthält.

**8.** Verfahren nach einem der Ansprüche 2 bis 5, dadurch gekennzeichnet, daß die eingesetzte Dicarbonsäure der Formel (I) entspricht, in der zwei Nachbaratome der linearen Kette des Restes R zu einem ungesättigten oder aromatischen, heterocyclischen oder carbocyclischen Ring gehören.

**9.** Verfahren nach einem der Ansprüche 1 bis 8, dadurch gekennzeichnet, daß die eingesetzte Dicarbonsäure der Formel (I) aus den folgenden Dicarbonsäuren ausgewählt ist:

- Adipinsäure,
- 2-Methyladipinsäure,
- 3-Methyladipinsäure,
- 4-Methyladipinsäure,
- 5-Methyladipinsäure,
- 2,2-Dimethyladipinsaure,
- 3,3-Dimethyladipinsäure,
- 2,2,5-Trimethyladipinsäure,
- 2,5-Dimethyladipinsäure,
- Pimelinsäure (Heptandisäure),
- 2-Methylpimelinsäure,
- 2,2-Dimethylpinlelinsäure,
- 3,3-Dimethylpimelinsäure,
- 2,5-Dimethylpimelinsäure,
- 2,2,5-Trimethylpimelinsäure,
- Azelainsäure
- Sebacinsäure und
- 1,2-Phenylendiessigsäure.

**10.** Verfahren nach einem der Ansprüche 1 bis 9, dadurch gekennzeichnet, daß die eingesetzte Dicarbonsäure der Formel (I) Adipinsäure oder 2,2-Dimethyladipinsäure ist.

**11.** Verfahren nach einem der Ansprüche 1 bis 10, dadurch gekennzeichnet, daß der eingesetzte Katalysator ein Phosphat, Pyrophosphat oder Polyphosphat ist.

**12.** Verfahren nach Anspruch 11, dadurch gekennzeichnet, daß das Gegenion ein Metallkation ist, vorzugsweise ein Element aus der Gruppe 1a, 2a oder 3b des Periodensystems der Elemente oder ein Ammoniumkation.

**13.** Verfahren nach einem der Ansprüche 1 bis 12, dadurch gekennzeichnet, daß der eingesetzte Katalysator ausgewählt ist aus Natrium- oder Kaliumphosphaten, -pyrophosphaten, -tripolyphosphaten oder -pentapolyphosphaten.

**14.** Verfahren nach einem der Ansprüche 1 bis 13, dadurch gekennzeichnet, daß die Reaktion in flüssiger Phase in Gegenwart eines Reaktionslösungsmittels durchgeführt wird.

**15.** Verfahren nach Anspruch 14, dadurch gekennzeichnet, daß das Reaktionslösungsmittel ausgewählt ist aus:

- aliphatischen und/oder aromatischen Kohlenwasserstoffen, insbesondere den Paraffinen wie insbesondere Dekan, Undekan, Dodekan oder Tetradekan; aromatischen Kohlenwasserstoffen wie insbesondere den Xylolen, Cumol und Kohlenwasserstofffraktionen aus einer Mischung von Alkylbenzolen, insbesondere Fraktionen von, Solvesso®-Typ,
- schweren Estern von anorganischen Säuren (z.B. Trikresylphosphat) oder von Carbonsäuren (z.B. Octylphthalat),
- Ethern, insbesondere aromatischen Ethern wie Biphenyloxid und/oder Benzyloxid,
- Paraffin- und/oder Naphthenölen sowie Erdöldestillationsrückständen.

**16.** Verfahren nach einen, der Ansprüche 1 bis 15, dadurch gekennzeichnet, daß die Konzentration an Dicarbonsäure in der Reaktionsmasse 20 bis 50 Gew.-% der Reaktionsmasse darstellt.

**17.** Verfahren nach einem der Ansprüche 1 bis 16, dadurch gekennzeichnet, daß der eingesetzte Katalysator, berechnet als Anzahl der Metallkationatome auf 100 Mol Dicarbonsäure, zwischen 0,1 und 20 %, vorzugsweise zwischen 1 und 10 %, liegt.

**18.** Verfahren nach einem der Ansprüche 1 bis 17, dadurch gekennzeichnet, daß man zunächst das Reaktionslösungsmittel und den Katalysator vorlegt und anschließend die Dicarbonsäure hinzugibt, die vorzugsweise zuvor aufgeschmolzen worden ist.

**19.** Verfahren nach einem der Ansprüche 1 bis 18, dadurch gekennzeichnet, daß die Zufuhrmenge an Dicarbonsäure pro Zeit zwischen 0,1 und 4,0 kg/h pro ein Kilogramm des zugegebenen Katalysators variiert, vorzugsweise zwischen 0,5 und 1,0 kg/h pro Kilogramm des Katalysators.

**20.** Verfahren nach einen, der Ansprüche 1 bis 19, dadurch gekennzeichnet, daß die Reaktionstemperatur zwischen 200 °C und 300 °C, vorzugsweise zwischen 250 °C und 290 °C, liegt.